# EUROPEAN PATENT SPECIFICATION

(11) **EP 3 862 009 B1**
(45) Date of publication and mention of the grant of the patent: **05.08.2026**
(21) Application number: 19869495.2
(22) Date of filing: 19.09.2019
(51) Int. Cl.: A61K 35/36, A61K 35/35, C12N 13/00, C12N 5/077

(54) **ULTRASOUND-INDUCED EXOSOMES OBTAINED FROM HUMAN DERMAL FIBROBLASTS, FOR USE IN WOUND HEALING**
DURCH ULTRASCHALL INDUZIERTE EXOSOMEN, GEWONNEN AUS MENSCHLICHEN DERMALEN FIBROBLASTEN, ZUR VERWENDUNG BEI DER WUNDHEILUNG
EXOSOMES INDUITS PAR ULTRASONS OBTENUS À PARTIR DE FIBROBLASTES DERMIQUES HUMAINS, POUR UNE UTILISATION DANS LA CICATRISATION DES PLAIES

(30) Priority: 02.10.2018 KR 20180117264
(43) Date of publication of application: 11.08.2021
(73) Proprietor: STEMON Inc., Gyeonggi-do 13486 (KR)
(72) Inventor: LEE, Yong Seung, Suwon-si Gyeonggi-do 16591 (KR)
(74) Representative: M. Zardi & Co S.A.
(86) International application number: PCT/KR2019/012146
(87) International publication number: WO 2020/071663

(56) References cited:
- WO-A1-2017/123022
- KR-A- 20140 066 456
- KR-A- 20170 106 149
- US-A1- 2018 177 828
- GEIGER ADOLF ET AL: "Human fibrocyte-derived exosomes accelerate wound healing in genetically diabetic mice", BIOCHEMICAL AND BIOPHYSICAL RESEARCH COMMUNICATIONS, vol. 467, no. 2, 1 November 2015 (2015-11-01), Amsterdam NL, pages 303 - 309, XP055848312, ISSN: 0006-291X, DOI: 10.1016/j.bbrc.2015.09.166
- YONG SEUNG LEE ET AL: "Exosome-Mediated Ultra-Effective Direct Conversion of Human Fibroblasts into Neural Progenitor-like Cells", ACS NANO, vol. 12, no. 3, 20 February 2018 (2018-02-20), US, pages 2531 - 2538, XP055604604, ISSN: 1936-0851, DOI: 10.1021/acsnano.7b08297
- HU , L.: "Exosomes derived from human adipose mensenchymal stem cells accelerates cutaneous wound healing via optimizing the characteristics of fibroblasts", SCIENTIFIC REPORTS, vol. 6, 12 September 2016 (2016-09-12), pages 32993, XP055485849
- SHABBIR, A.: "Mesenchymal stem cell exosomes induce proliferation and migration of normal and chronic wound fibroblasts, and enhance angiogenesis in vitro", STEM CELLS AND DEVELOPMENT, vol. 24, 2015, pages 1635 - 1647, XP055583096, DOI: 10.1089/scd.2014.0316
- ZHOU, S.: "Molecular mechanisms of low intensity pulsed ultrasound in human skin fibroblasts", JOURNAL OF BIOLOGICAL CHEMISTRY, vol. 279, no. 52, 12 October 2004 (2004-10-12), pages 54463 - 54469, XP055704108

## Description

### [Technical Field]

The present invention relates to exosomes having wound healing effects.

### [Background Art]

The skin, the human body's largest organ, basically has an important function as a primary line of defense that provides protection against diseases and water evaporation, in addition to various functions of receiving sensations, synthesizing vitamin D and participating in body temperature regulation and immune surveillance. Skin wound healing is a complicated process that involves four stages: hemostasis, inflammation, proliferation, and remodeling. In the first stage that occurs from immediately after wound formation to within a few hours, blood clotting occurs and a provisional wound matrix is formed, and in the inflammation stage activated through the first stage, infectious agents and necrotic tissue are removed through neutrophil recruitment and monocyte recruitment and degeneration. In the proliferation stage, granulation tissue formation, vascular network restoration, migration to the surface of fibroblasts, reepithelization, angiogenesis, etc. occur, and in the remodeling stage that continues from the 21^{st} day to 1 year after wound formation, the processes actively progressing in the proliferation stage step slowly, such as stopping of the formation of granulation tissue through apoptosis, and a large amount of collagen III produced in the proliferation stage is replaced by tougher collagen I, and myofibroblasts are connected to collagen, reducing the scar area. If even any one of the above processes does not work properly, healing of the wound may be delayed or the wound become chronic. In this case, in particular, serious problems in the role of the primary line of defense may arise, or otherwise, external problems may occur due to scar hyperplasia, etc. even if healing is achieved.

Skin appendages include hair roots, sebaceous glands, nails, and sweat glands. Healing of large wounds differs from species to species. In the case of lower vertebrates such as fish and amphibians, complete regeneration of the skin including skin appendages, such as scales and glands, occurs. On the other hand, in mammals except for the embryonic stage, skin regeneration is not complete, and hence scar tissue without skin appendages is created, which prevents the functions of skin appendages related to additional protection, warmth, and sensation from being properly performed and lowers the quality of life in terms of beauty and psychology.

Biologics, which are pharmaceutical drugs based on proteins, genes, cells, etc. derived from organisms, have advantages in that they have low toxicity because they produce no metabolite *in vivo,* unlike synthetic drugs, exhibit good effects by selectively acting on the pathogenesis of diseases, and have fewer side effects. In recent years, as these advantages have attracted attention, methods that use biologics, particularly stem cells, have been developed for the purpose of skin regeneration and wound healing, and methods that use stem cells themselves, or extracts or conditioned media thereof, have been disclosed. However, the compositions using stem cells commonly have problems in terms of efficiency and economy due to complicated stem cell isolation and multiplication, and the stem cell therapy product has a disadvantage in that it is cost-ineffective and time-consuming because it is not easy to purify an active ingredient from cells. In addition, the stem cell-conditioned medium has a problem in that it contains wastes secreted from cells or substances that have not been verified for safety in human use, such as antibiotics added to prevent contamination and serum, in addition to containing an active ingredient.

It is known that exosomes are naturally secreted nanovesicles having a diameter of 30 to 200 nm, and can act as important nanomediators that deliver various substances from cells. Since the discovery that exosomes can change the phenotype of target cells through mRNA delivery, several studies have revealed that exosomes are involved in cell differentiation and the like. In recent years, the possibility of applying exosomes directly to the human body for therapeutic purposes has been explored in various ways. In particular, exosomes may exhibit more complex and long-lasting effects in the biologics field, because they contain a variety of proteins and nucleic acids compared to biopharmaceuticals such as small molecules, peptides, growth factors, antibodies, nucleic acids, etc. In addition, exosomes do not cause risk of development into cancer, unlike large-sized medicines such as cells, and may advantageously have spatiotemporal effects different from those of cells because they do not require degradation into vesicles, which occurs when cells are injected. In recent years, in particular, technology of using exosomes, obtained from stem cells known to have effects of ameliorating and treating various symptoms, as therapeutic compositions, has been developed. Korean Patent Application Publication No. 10-2017-0044999 (October 16, 2015) discloses a composition for wound healing containing, as an active ingredient, exosomes derived from adult stem cells or a conditioned medium thereof.

The patent application US 2018/177828 A1 discloses stem cell exosomes having increased levels of heat shock protein for use in skin wound healing. The patent application WO 2017/123022 discloses exosomes having an increased content in growth factor e.g. VEGF, EGF or FGF, for improving the skin condition. The publication BIOCHEMICAL AND BIOPHYSICAL RESEARCH COMMUNICATIONS, vol. 467, no. 2, 1 November 2015 (2015-11-01), pages 303-309 discloses human fibrocyte-derived exosomes for use in wound healing in diabetic mice. The publication *"*Exosome-Mediated Ultra-Effective Direct Conversion of Human Fibroblasts into Neural Progenitor-like Cells", ACS NANO, vol. 12, no. 3, 20 February 2018 (2018-02-20), pages 2531-2538, discloses that ultrasound treatment of human dermal fibroblasts activates the production of exosomes by the cells. The publication *"*Exosomes derived from human adipose mensenchymal stem cells accelerates cutaneous wound healing via optimizing the characteristics of fibroblasts". Scientific reports, vol. 6, 12 September 2016, page 32993, discloses that exosomes derived from adipose mesenchymal stem cells accelerate cutaneous wound healing. The publication *"*Mesenchymal stem cell exosomes induce proliferation and migration of normal and chronic wound fibroblasts, and enhance angiogenesis in vitro", Stem cells and development, vol. 24, 2015, pages 1635-1647, discloses that exosomes from mesenchymal stem cells enhance normal and diabetic wound fibroblast migration and induce angiogenesis.

However, in technologies related to therapeutic compositions using exosomes, including the technology of the above-described patent application publication, exosomes are obtained in low yield, and these technologies mostly use stem cells that are difficult to isolate and multiply. Therefore, in order to use exosomes clinically, there is a need for a technology capable of increasing the yield of exosomes while obtaining the exosomes from cells that may be easily obtained, multiplied and maintained, not from stem cells.

### [Disclosure]

### [Technical Problem]

The present invention has been made in order to solve the above-described problems, and the present invention provides exosomes for use in wound healing, obtained in high yield within a short time from cells from which the exosomes are easily obtainable; the exosome preparation method, which is defined by claim 1, comprises steps of: providing ultrasound stimulation directly or indirectly to cells; culturing a mixture of the cells and a medium for a predetermined time; and isolating exosomes from the mixture, wherein providing the stimulation directly to the cells comprises applying ultrasound stimulation to a medium containing the cells, and providing the stimulation indirectly to the cells comprises applying ultrasound stimulation a medium not containing the cells and then mixing the medium and the cells.

The present invention provides exosomes produced by the above production method, which have increased RNA of a gene that promotes skin generation and wound healing, for use in wound healing.

### [Technical Solution]

As a technical means for achieving the above-described technical problem, exosomes produced in accordance with claim 1 are herein presented for use in wound healing. The method for producing the exosomes is defined by claim 1. The method is based on providing ultrasound stimulation directly or indirectly to cells; culturing a mixture of the cells and a medium for a predetermined time; and isolating exosomes from the mixture, wherein providing the stimulation directly to the cells comprises applying ultrasound stimulation to a medium containing the cells, and providing the stimulation indirectly to the cells comprises applying ultrasound stimulation to a medium not containing the cells and then mixing the medium and the cells.

Here, the step of providing ultrasound stimulation directly or indirectly to cells is performed, in accordance with claim 1, by providing ultrasound stimulation to each of the cells and a medium and then mixing the cells and the medium.

The medium is any one of epithelial stem cell medium, dermal stem cell medium, adipose stem cell medium, mesenchymal stem cell medium, or embryonic stem cell medium. The culturing of the mixture is performed for 1 hour to 10 days.

The step of isolating the exosomes may be performed using one or more of ultracentrifugation, density gradient separation, filtration, size exclusion chromatography, immunoaffinity separation, precipitation, and microfluidic separation.

The step of isolating the exosomes comprises steps of: centrifuging the mixture after the culturing to obtain a supernatant; filtering the supematant through a filter to obtain a filtrate; and concentrating the filtrate.

The step of isolating the exosomes may further comprise a step of storing the supernatant at 4°C or below for 7 days to 1 month before filtering the supematant through the filter.

The isolated exosomes may have a diameter of 50 to 200 nm.

Here, the exosomes may have increased RNA of a gene that promotes skin regeneration and wound healing, compared to exosomes secreted from the cells before being subjected to the method of claim 1, wherein the gene that promotes skin regeneration and wound healing may be any one or more of EGFR, FN1, MMP9, MMP2, MAPK1, FGF2, CTGF, AKT1, ALB, TGFB1, MAPK3, VEGFA, FGF7, HGF, IL6, EGF, miR-210, miR-31, Col1α1, ELN, PCNA, N-cadherin and cyclin-D1.

The exosomes, when applied to the skin, may further increase RNA expression of a gene that promotes skin regeneration and wound healing in the skin, compared to exosome secreted from the cells before being subjected to the method of claim 1, wherein the gene that promotes skin regeneration and wound healing may be any one or more of Col1α1, Col3α1, ELN, PCNA, N-cadherin and cyclin-D1.

A composition containing the above-described exosomes may contain the exosomes at a concentration of 10⁶ exosomes/ml or more.

### [Advantageous Effects]

The method for producing exosomes defined by claim 1 may induce secretion of a large amount of exosomes having skin regeneration and wound healing effects not only from stem cells and progenitor cells that are difficult to isolate and multiply, but also from easily available somatic cells, within a short time by ultrasonic treatment that is a simple process. The exosomes thus induced are obtained in higher yield than those in a conventional method, and the amount and number of various factors contained in the exosomes are also large.

The exosomes for use according to claim 1 and a composition containing the same contain large amounts of various factors capable of inducing skin regeneration and wound healing. In addition, the exosomes according to one embodiment of the present invention have a phospholipid-based membrane structure, and thus may easily penetrate the pores and deliver substances with high efficiency, thus effectively inducing skin regeneration and wound healing. Moreover, the exosomes have the effect of having no side effects, which occur due to the use of synthetic drugs, and advantageously have a longer-lasting effect. In addition, the exosomes according to one embodiment of the present invention allow wound healing (which is generally incomplete in mammals) to be more complete, thus making sure no scars remain or making the scars smaller in size, and may also regenerate skin appendages, such as hair roots at the wound site.

### [Description of Drawings]

FIG. 1 shows data related to the production of exosomes having wound healing effects according to Example 1 of the present invention, and depicts immunofluorescence staining images showing induction of the exosome marker CD63.
FIG. 2 shows the data of gene expression in the exosomes according to Example 1 of the present invention, and shows the results of gene ontology analysis of genes having 12 or more read counts in the exosomes (iExo) by RNA-seq.
FIG. 3 shows the data of gene expression in the exosomes according to Example 1 of the present invention, and shows the results of RNA-seq of wound healing related genes.
FIG. 4 shows the data of gene expression in the exosomes according to Example 1 of the present invention, and shows the results of RNA-seq related to angiogenesis, cell growth, cell migration, cell proliferation, inflammatory response, regeneration, secretion, tissue regeneration, and tissue remodeling.
FIG. 5 shows the data of gene expression in the according to Example 1 of the present invention, and shows qRT-PCR data obtained by analyzing the RNAs of skin regeneration- and wound healing-related genes in the exosomes.
FIG. 6 shows data related to the *in vitro* effects of the exosomes according to Example 1 of the present invention, and shows data obtained by analyzing the proliferation of HDF cells treated with various concentrations of the exosomes.
FIG. 7 shows data related to the *in vitro* effects of the exosomes according to Example 1 of the present invention, and shows the results of measuring the migration rate of HDFs, treated with various concentrations of the exosomes, by scratch assay.
FIG. 8 shows data related to the *in vitro* effects of the exosomes according to Example 1 of the present invention, and shows data obtained by analyzing tube formation of HUVECs treated with various concentrations of the exosomes.
FIG. 9 shows data related to the *in vitro* effects of the exosomes according to Example 1 of the present invention, and shows qRT-PCR data obtained by analyzing the RNAs of skin regeneration- and wound healing-related genes in cells treated with the exosomes.
FIG. 10 shows data related to the *in vivo* effects of the exosomes according to Example 1 of the present invention, and depicts showing the time-dependent wound healing process in mice treated with various concentrations of the exosomes.
FIG. 11 shows data related to the *in vivo* effects of the exosomes according to Example 1 of the present invention, and depicts H&E staining images of wound site tissue sections in mice treated with various concentrations of the exosomes.
FIG. 12 shows data related to the *in vivo* effects of the exosomes according to Example 1 of the present invention, and shows data obtained by analyzing a cell proliferation marker in wound sites in mice treated with various concentrations of the exosomes.
FIG. 13 shows data related to the *in vivo* effects of the exosomes according to Example 1 of the present invention, and shows data obtained by analyzing skin penetration of the exosomes.
FIG. 14 shows data related to the *in vivo* effects of the exosomes according to Example 1 of the present invention, and shows the results of RT-PCR for genes in wound sites in mice treated with various concentrations of the exosomes.
FIG. 15 shows data related to the *in vivo* effects of the exosomes according to Example 1 of the present invention, and shows images comparing the development of subcutaneous blood vessels in the wound site 3 weeks after wound formation in mice treated with the exosomes.

### [Best Mode]

Hereinafter, the present invention will be described in more detail. However, the scope of the present invention should be defined only by the appended claims.

The terminology used herein is only for the purpose of describing particular embodiments and is not intended to be limiting of the present invention. Singular expressions include plural expressions unless otherwise specified in the context thereof. Throughout the present specification, it is to be understood that when any part is referred to as "comprising" any component, it does not exclude other components, but may further comprise other components, unless otherwise specified.

A method for producing exosomes according to claim 1 is based on: providing ultrasound stimulation directly or indirectly to cells; culturing a mixture of the cells and a medium for a predetermined time; and isolating exosomes from the mixture, wherein providing the stimulation directly to the cells comprises applying ultrasound stimulation to the medium containing the cells, and providing the stimulation indirectly to the cells comprises applying ultrasound stimulation to the medium not containing the cells and then mixing the medium and the cells.

Here, the step of providing ultrasound stimulation directly or indirectly to cells is performed by providing ultrasound stimulation to each of the cells and the medium and then mixing the cells and the medium. The ultrasound stimulation may be provided directly or indirectly to the cells one or more times, and as the number of times increases, the wound healing effects of exosomes may increase proportionally. When the ultrasound stimulation is provided one or more times as described above, it is preferable to provide a time interval between the provisions so that the cells can recover, and the time interval may be at least 1 day, more preferably at least 2 days.

The cells may be selected in accordance with claim 1..

The step of providing ultrasound stimulation to the cells may be performed either by directly treating the cells with ultrasound or in a state in which only a minimum amount of the cells are contained in an initial culture medium so that the cells barely covers the initial culture medium. Here, the initial culture medium is a common medium used to maintain the cells in a healthy state, and may be a medium suitable for normal culture of the cells. For example, the cells may be fibroblasts, and the initial culture medium may be DMEM medium containing antibiotics and serum.

The culture medium (a medium that is treated with ultrasound without cells) may be selected from among epithelial stem cell medium, dermal stem cell medium, adipose stem cell medium, mesenchymal stem cell medium, or embryonic stem cell medium. For example, the culture medium may be any one of skin stem cell medium, bone marrow stem cell medium, vascular endothelial progenitor cell medium, vascular progenitor cell medium, mesenchymal progenitor cell medium, epithelial stem cell medium, dermal stem cell medium, hematopoietic stem cell medium, adipose stem cell medium, mesenchymal stem cell medium, embryonic stem cell medium, and various adult stem cell media. The invention of Korean Patent Application No. 10-2018-0060317 filed in the name of present inventor discloses that, when exosomes are obtained by culturing a mixture of an ultrasound-treated second cell culture medium (a medium capable of culturing second cells or inducing differentiation into second cells) and ultrasound-treated first cells and other cells are treated with the exosomes, the cells may be reprogrammed into the second cells, and that stem cells or extracts or conditioned media thereof all exhibit an effect on skin regeneration and wound healing.

The culturing of the mixture is performed for 1 day.

The step of isolating the exosomes may be performed using one or more of ultracentrifugation, density gradient separation, filtration, size exclusion chromatography, immunoaffinity separation, precipitation, and microfluidic separation.

The step of isolating the exosomes may comprise steps of: centrifuging the mixture after the culturing to obtain a supernatant; filtering the supematant through a filter to obtain a filtrate; and concentrating the filtrate. The centrifugation is performed to remove cell debris and dead cells, and may preferably be performed at 1,000 to 5,000 g for 10 to 60 minutes. The step of filtering the supematant through the filter is performed to further remove cellular debris and obtain only particles having a specific size or less, and the filter used herein may be preferably a syringe filter. The step of concentrating the filtrate may preferably be performed using a centrifugal filter. Where the centrifugal filter is used, it is possible to remove particles having a specific size or less while concentrating the filtrate. The step of isolating the exosomes may further comprise a step of storing the supematant at 4°C or below for 7 days to 3 months before filtering the supernatant through the filter. The storage may preferably be done at 4°C or less for 7 days or less, more preferably -20°C or less for 1 month or less, most preferably -80°C or less for 3 months or less. The active ingredients of the exosomes include mRNA and protein, and these ingredients may be more easily denatured or degraded as the temperature is higher or closer to the temperature at which the enzyme activity is high.

The isolated exosomes may have a diameter of 50 to 200 nm, preferably 100 to 150 nm.

Here, wound healing may include one or more of anti-aging, wrinkle prevention, wrinkle reduction, wound healing, scar healing, and tissue regeneration.

The exosomes may have increased RNA of a gene that promotes skin regeneration and wound healing, compared to exosomes secreted from the cells before being subjected to the above-described production method, wherein the gene that promotes skin regeneration and wound healing may be any one or more of EGFR (epidermal growth factor receptor), FN1 (fibronectin 1), MMP9 (matrix metallopeptidase 9), MMP2 (matrix metallopeptidase 2), MAPK1 (mitogen-activated protein kinase 1), FGF2 (fibroblast growth factor 2), CTGF (connective tissue growth factor), AKT1 (AKT serine/threonine kinase 1), ALB (albumin), TGFB1 (transforming growth factor beta 1), MAPK3 (mitogen-activated protein kinase 3), VEGFA (vascular endothelial growth factor A), FGF7 (fibroblast growth factor 7), HGF (hepatocyte growth factor), IL6 (interleukin 6), EGF (epidermal growth factor), miR-210 (MicroRNA 210), miR-31(MicroRNA 31), Col1α1 (collagen 1 alpha 1), ELN (elastin), PCNA (proliferatin cell nuclear antigen), N-cadherin, and cyclin-D1.

The exosomes, when applied to the skin, may further increase RNA expression of a gene that promotes skin regeneration and wound healing in the skin, compared to exosome secreted from the cells before being subjected to the above-described production method, wherein the gene that promotes skin regeneration and wound healing may be any one or more of Col1α1, Col3α1, ELN, PCNA, N-cadherin, and cyclin-D1.

Still another aspect of the present invention provides a composition containing the exosomes, for use in accordance with claim 1.

Here, the composition may contain the exosomes at a concentration of at least 10⁶ exosomes/ml, more preferably 10⁶ to 10¹⁴ exosomes/ml, more preferably 10¹¹ to 10¹² exosomes/ml. This is because if the concentration of exosomes in the composition is less than 10⁶ exosomes/ml or more than 10¹⁴ exosomes/ml, the wound healing effects may decrease, and in particular, if the concentration is excessively high, the economic efficiency may also decrease.

It is obvious that the composition may further contain a pharmaceutically acceptable carrier and/or other additives that can further enhance the uptake of active ingredients or healing responses, in addition to the exosomes, and thus detailed description of the pharmaceutically acceptable carrier and other additives will be omitted.

### [Mode for Invention]

Hereinafter, examples of the present invention will be described in detail so that the present invention can be easily carried out by those skilled in the art..

All cell culture processes in the Examples and Experimental Examples of the present invention were performed at 37°C under 5% CO₂.

### Example 1. Production of Exosomes Having Skin Regeneration and Wound Healing Effects

To obtain exosomes having skin regeneration and wound healing effects, ultrasound stimulation with 20 KHz and 1.0 W/cm² was applied directly to 1 x 10⁶ human dermal fibroblasts (HDFs) for 5 seconds by means of UltraRepro 1001 (STEMON Inc., Seoul, Republic of Korea). 2 x 10⁵ UHDFs (the HDFs to which ultrasound stimulation was applied) were cultured with an ultrasound-treated (at 20 KHz and 5.0 W/cm² for 10 min) embryonic stem cell medium (DMEM/F12, 15% FBS, 2 mM GlutaMAX, 0.1% NEAA, 0.1% penicillin/streptomycin, 0.1 mM β-mercaptoethanol, 1000 units/ml leukemia inhibitory factor (LIF)) in a 35-mm Petri dish for one day. Exosomes were isolated from the conditioned medium of the ultrasound-treated HDFs (UHDFs) as follows: The conditioned medium was centrifuged at 3,000 xg for 20 minutes to remove cell debris and dead cells, and then the supematant was passed through a 0.22-mm filter (Minisart^{®}Syringe Filter, Sartorius, Goettingen, Germany). The medium that passed through the filter was placed in Amicon^{®}Ultra-15 100,000 kDa device (Millipore, Billerica, MA, USA) and centrifuged at 14,000 xg for 20 minutes to concentrate exosomes (iExo).

### Experimental Example 1. Experiment for Analysis of Exosomes Having Skin Regeneration and Wound Healing Effects

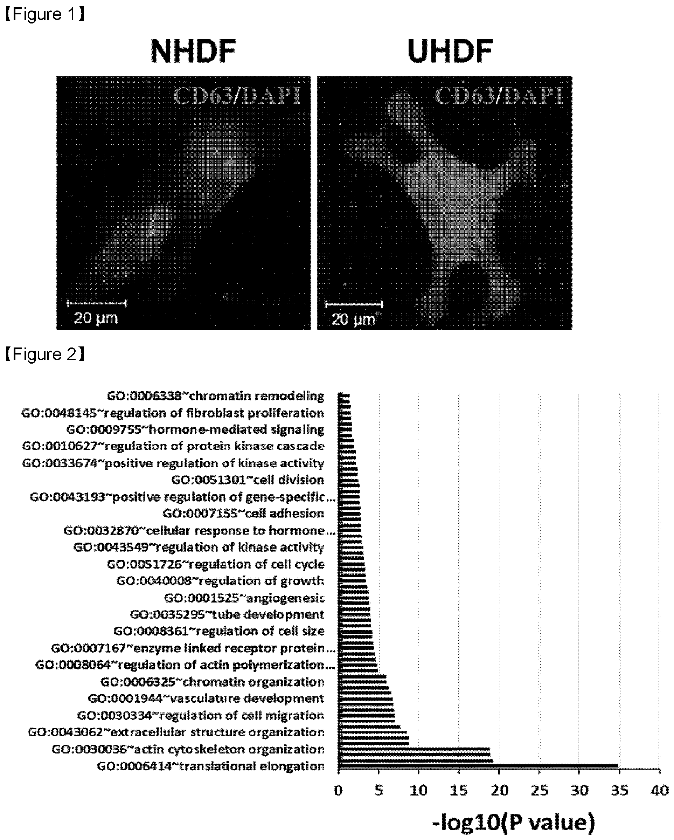

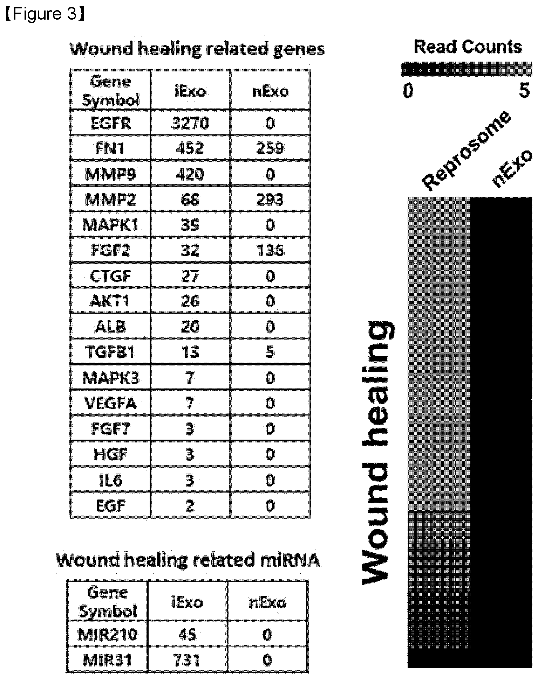

The UHDFs cultured according to the method of Example 1 for inducing exosomes having skin regeneration and wound healing effects were analyzed through immunofluorescence staining for the CD63 exosome marker (at this time, counter staining was performed using the nuclear staining dye DAPI). As a result, it could be confirmed that the CD63 signal intensity in the UHDFs was significantly higher than that in the control group, suggesting that a large amount of exosomes were produced in the UHDFs (FIG. 1). As a result of RNA-Seq analysis for the produced exosomes, it was shown that expression of particularly genes related to wound healing, angiogenesis, cell growth, cell migration, cell proliferation, inflammatory response, regeneration, secretion, tissue regeneration, and tissue remodeling, was significantly higher in the exosomes (iExo) produced according to Example 1 than in exosomes (nExo) isolated from cells cultured without ultrasound stimulation (FIGS. 2 to 4), the genes typically including genes encoding proteins such as EGFR, FN1, MMP9, MMP2, MAPK1, FGF2, CTGF, AKT1, ALB, TGFB1, MAPK3, VEGFA, FGF7, HGF, IL6, and EGF, and microRNAs such as miR-210 and miR-31 (FIG. 3).

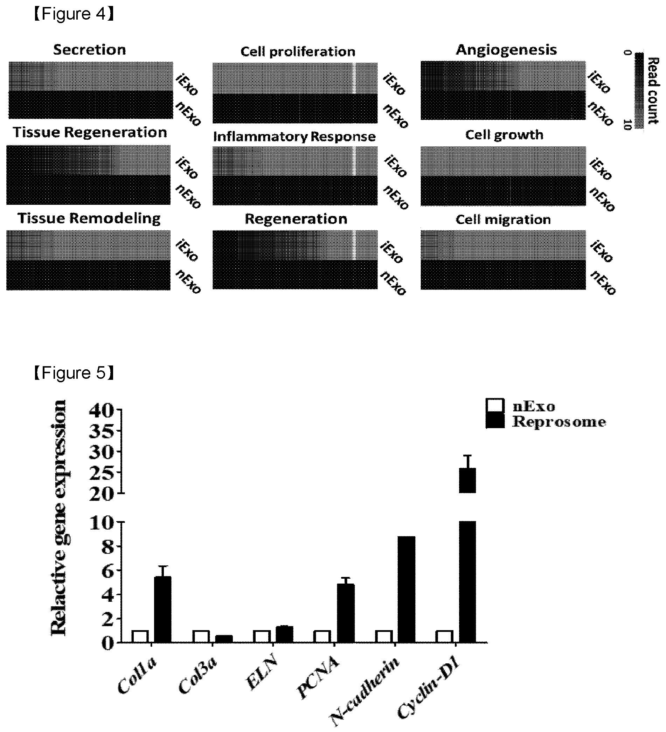

In addition, RNA was extracted from the exosomes (iExo) isolated from the conditioned medium of the cells and was analyzed by qRT-PCR. As a result, it could be confirmed that expression of Col1α1, Col3α1 and ELN, which are extracellular matrix-related genes involved in tissue regeneration and wound healing, and PCNA, N-cadherin and cyclin-D1, which are cell proliferation-related genes, was higher in the exosomes (iExo) than in the exosomes (nExo) isolated from the control group (FIG. 5).

### Experimental Example 2. Experiment for Analysis of Phenotypes of Cells Treated with Exosomes Having Skin Regeneration and Wound Healing Effects

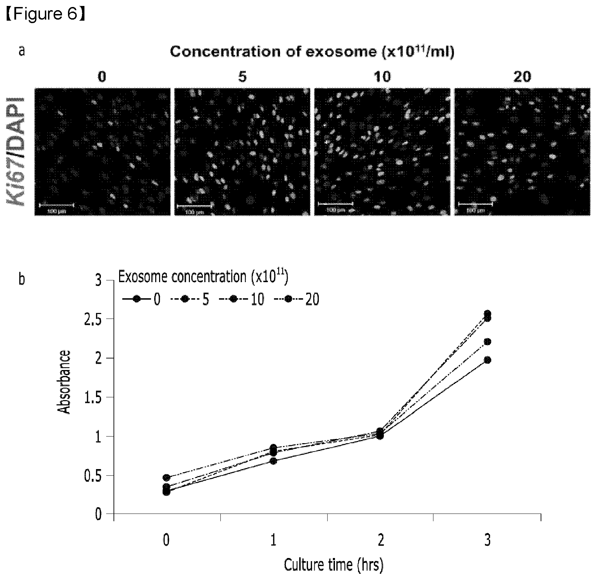

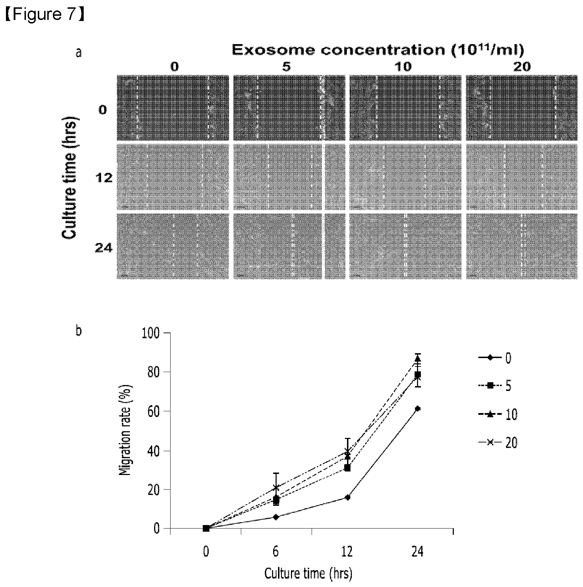

The exosomes produced according to the method of Example 1 for producing exosomes having skin regeneration and wound healing effects were added to medium, and HDFs were cultured in the medium. Then, concentration-dependent effects of the endosomes were analyzed. First, as a result of analyzing the cell proliferation effect of the exosomes through immunofluorescence staining for Ki67, a related marker, it could be confirmed that expression of the marker was higher in the experimental group than in the control group, and particularly when the cells were treated with the exosomes at concentrations of 5 x 10¹¹ exosomes/ml and 10 x 10 ¹¹ exosomes/ml, the effect of the exosomes was better and the number of the cells increased more (FIG. 6). The HDFs were cultured to fill the plate, and then wound healing assay was performed by creating lines by a 20 µl yellow tip to increase the spacing between the cells, and then treating the cells with the exosomes, followed by culture. As a result, it was confirmed that the empty space in the exosome-treated group was filled relatively quickly (FIG. 7), suggesting that the migration rate of the cells in the exosome-treated group was higher.

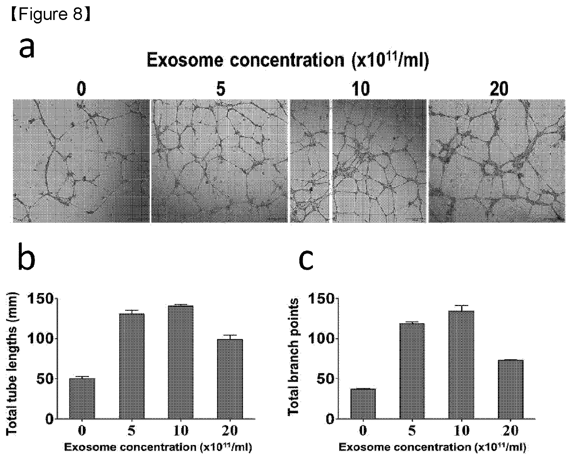

Next, in order to analyze angiogenesis efficiency during tissue regeneration depending on the concentration of the exosomes produced according to Example 1, endothelial cells (HUVECs) were treated with different concentrations of the exosomes, and after 10 hours, tube formation was checked. As a result, it could be visually confirmed that the exosome-treated groups easily formed tubes compared to the control group (FIG. 8a), and the tube length and the number of branch points were also higher in the exosome-treated groups (FIGS. 8b and 8c), particularly higher in the groups treated with the exosomes at concentrations of 5 x 10¹¹ exosomes/ml and 10 x 10¹¹ exosomes/ml.

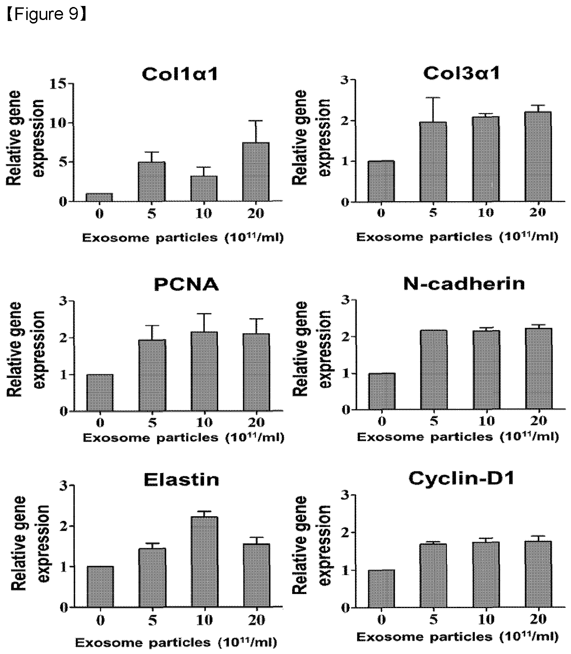

Finally, expression of Col1α1, Col3α1, ELN, PCNA, N-cadherin and cyclin-D1, which are tissue regeneration- and wound healing-related genes, in the exosome-treated cells, was analyzed by qRT-PCR. As a result, it was confirmed that expression of the wound healing-related genes in the exosome-treated group was higher than that in the control group (FIG. 9).

### Experimental Example 3. Experiment for Analysis of In Vivo Effect of Treatment with Exosomes Having Skin Regeneration and Wound Healing Effects

In order to analyze the *in vivo* effect of the exosomes (iExo) produced according to the method of Example 1 for producing exosomes having skin regeneration and wound healing effects, a 6-mm wound was created on the skin of each 6-week-old C57 mouse using a punch, and iExo were diluted in PBS at concentrations of 0, 5, 10 and 20 x 10¹¹/ml and applied to each wound site twice a week.

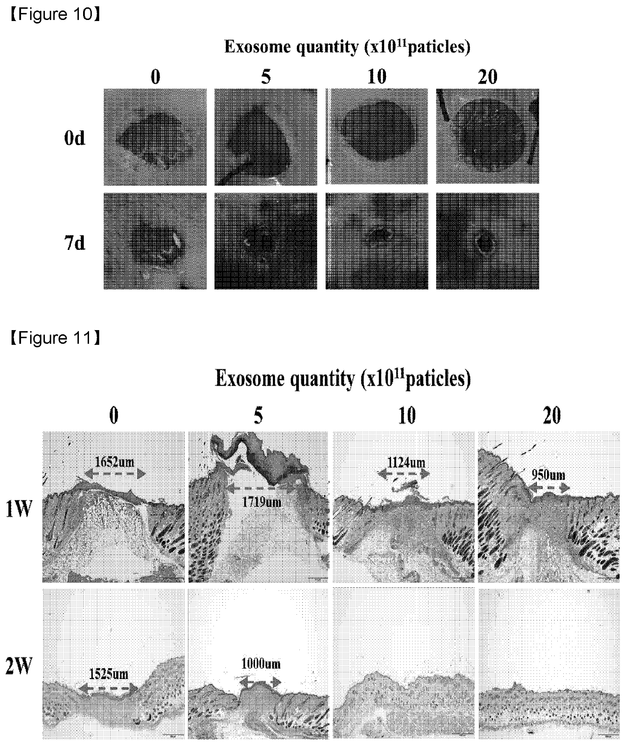

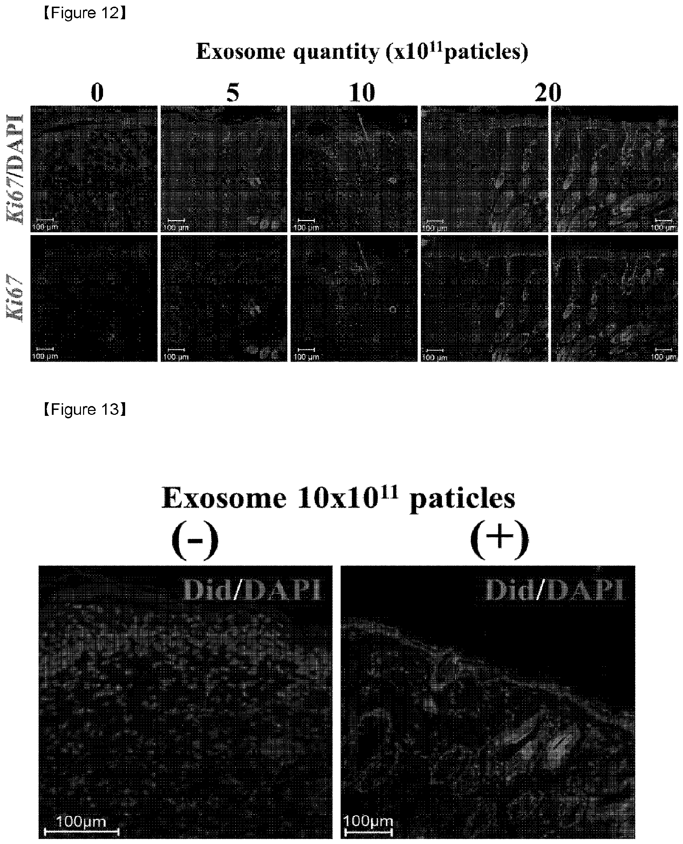

As a result, it could be confirmed that the size of the externally exposed wound (opening) in the exosome-treated mice after 1 week significantly decreased compared to that in the untreated mice (FIG. 10; in this regard, the wound in the exosome-treated mice at week 2 was completely closed and the hair grew over the wound, and thus it was not possible to take a comparative picture between the treated groups). In addition, after 1 week and 2 weeks, the tissue section of each wound site was analyzed by H & E staining. As a result, it could be confirmed that the wound size in the exosome-treated mice was smaller than that in the non-exosome-treated mice, and thus skin regeneration and wound healing occurred more quickly in the exosome-treated mice. In particular, when the wound site was treated with the exosomes at a concentration of 10 or 20 x 10¹¹ exosomes/ml, the skin tissue was completely recovered at week 2, and thus the epidermis, dermal layer, fat layer and muscle layer constituting the skin tissue were regenerated so that they could be clearly distinguished from one another. In addition, in this case, hair follicles, which are skin appendages, were located in the dermal layer and the fat layer without any difference between the wound site and the surrounding area. In particular, it was confirmed that, when the wound site was treated with the exosomes at a concentration of 10 or 20 x 10¹¹ exosomes/ml, even the scar disappeared at week 2 (FIG. 11). Additionally, as a result of analyzing the cell proliferation marker Ki67 in the tissue section of the site recovered from the wound through immunofluorescence staining, it could be confirmed that Ki67 was more extensively observed in the exosome-treated group than in the non-exosome-treated group (FIG. 12), indicating that cell proliferation actively occurred at the wound site. iExo were stained with Vybrant^{™}DiD cell-labeling solution and the wound site was treated with the stained iExo, and after 2 weeks, the iExo-treated site was analyzed under a fluorescence microscope. As a result, it could be confirmed that the iExo penetrated the entire wound site, and the above-described regeneration and healing effects could appear due to this effective penetration (FIG. 13).

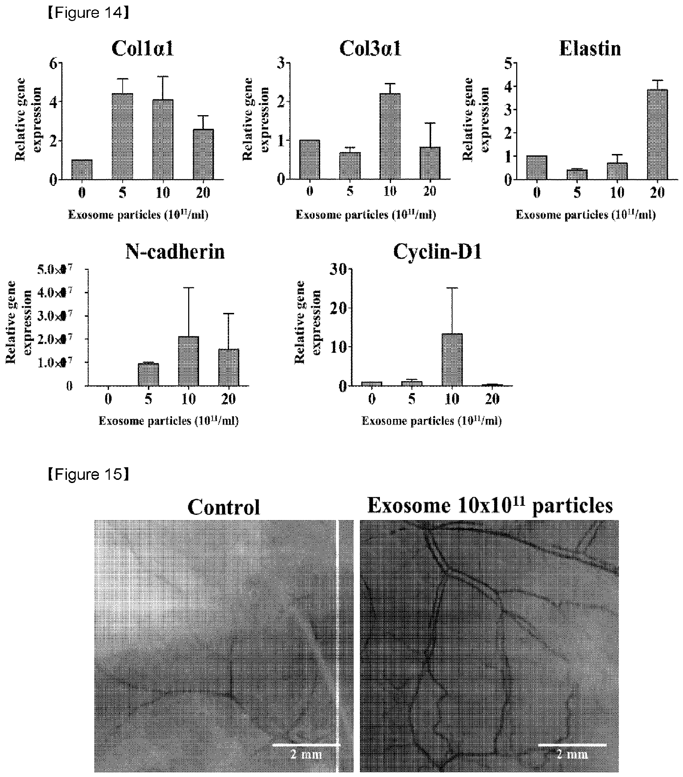

Next, expression of the tissue regeneration- and wound healing-related genes Col1α1, Col3α1, ELN, N-cadherin and cyclin-D1 in the wound site treated with the exosomes was analyzed by qRT-PCR, and as a result, it was confirmed that expression of one or more of the genes in the experimental group treated with iExo was generally higher than that in the control group (FIG. 14). At this time, the gene with the greatest increase in expression was different between the exosome concentrations, and it was difficult to confirm the correlation between the concentration and the increase or decrease in expression.

Finally, at week 3 when wound healing was completed, the degree of development of subcutaneous blood vessels was analyzed. As a result, it was confirmed that blood vessels in the tissue treated with iExo more developed (FIG. 15).

Taken together, the exosomes produced according to Example 1 can improve the wound healing and regeneration ability of tissue by promoting cell proliferation and migration and blood vessel development. In addition, since the effect of the exosomes also appears *in vivo,* the exosomes may increase the rate of skin regeneration and wound healing, and may also induce more complete skin regeneration and wound healing so that skin appendages may be regenerated with minimized scars or without scars.

### Comparative Example 1. Control for Exosomes

Non-ultrasound-treated HDFs (NHDFs) were cultured in a fibroblast medium (10% fetal bovine serum (Gibco)) containing 2 mM GlutaMAX^{™}-I supplement (Gibco), 6 U/mL heparin (Sigma-Aldrich) and 200 µM ascorbic acid (Sigma-Aldrich), and DMEM (Gibco) containing 1% penicillin/streptomycin (Gibco)). Isolation of exosomes (nExo) from the conditioned media of the NHDFs was performed in the same manner as the exosome isolation method of Example 1.

### Comparative Example 2. Control for Cells Treated with Exosomes

1 x 10⁵ HDFs were seeded and cultured in a 35-mm Petri dish for one day, and then the medium was replaced with a medium containing the exosomes obtained according to Comparative Example 1, followed by 5 days of culture. At this time, embryonic stem cell culture medium was used as the medium containing the exosomes, and the culture medium was replaced every 2 days.

### Comparative Example 3. Control for Mice in which Skin Regeneration and Wound Healing were Induced by Exosomes

The iExo dilution vehicle PBS was applied twice a week to mice with a punch wound of 6 mm in diameter.

### [Industrial Applicability]

The exosomes for use according to claim 1 and a composition containing the same contain large amounts of expression products of various factors capable of inducing skin regeneration and wound healing, particularly genes that promote skin regeneration and wound healing. In addition, the exosomes have a phospholipid-based membrane structure, and thus may easily penetrate the skin and deliver substances with high efficiency, thus effectively inducing skin regeneration and wound healing. Moreover, the exosomes do not have side effects occurring due to the use of synthetic drugs, have a longer-lasting effect, and may be used as a drug capable of inducing regeneration and healing when tissues including skin are damaged.

In addition, the method described in claim 1 proceeds in high yield through an easy process of treating cells with ultrasound stimulation.

## Claims

1. Exosomes for use in wound healing, wherein said exosomes are obtained from a method comprising steps of:
(a) applying ultrasound stimulation with intensity of 1 W/cm² at a frequency of 20 KHz for a duration of 5 seconds to a medium containing human dermal fibroblasts (HDF), the medium being any one of epithelial stem cell medium, dermal stem cell medium, adipose stem cell medium, mesenchymal stem cell medium, or embryonic stem cell medium; and applying ultrasound stimulation, with intensity of 5 W/cm² at a frequency of 20 KHz for a duration of 10 minutes, to an embryonic stem cell medium not containing the HDF;
(b) mixing the medium containing the HDF, obtained from step (a), with the medium not containing the HDF, obtained from step (a);
(c) culturing the mixture obtained from step (b) for 1 day; and isolating exosomes from the mixture.

## Patentansprüche

1. Exosomen zur Verwendung bei einer Wundheilung, wobei die Exosomen aus einem Verfahren erhalten werden, das die Schritte umfasst:
(a) Anlegen einer Ultraschallstimulation mit einer Intensität von 1 W/cm² bei einer Frequenz von 20 kHz für eine Dauer von 5 Sekunden an ein Medium, das humane dermale Fibroblasten (HDF) enthält, wobei das Medium eines von einem Epithelstammzellmedium, einem dermalen Stammzellmedium, einem adipösen Stammzellmedium, einem mesenchymalen Stammzellmedium oder einem embryonalen Stammzellmedium ist; und Anlegen einer Ultraschallstimulation mit einer Intensität von 5 W/cm² bei einer Frequenz von 20 kHz für eine Dauer von 10 Minuten an ein embryonales Stammzellmedium, das die HDF nicht enthält;
(b) Mischen des die HDF enthaltenden Mediums, das aus Schritt (a) erhalten wurde, mit dem die HDF nicht enthaltenden Medium, das aus Schritt (a) erhalten wurde;
(c) Kultivieren der aus Schritt (b) erhaltenen Mischung für 1 Tag; und Isolieren von Exosomen aus der Mischung.

## Revendications

1. Exosomes pour utilisation dans la cicatrisation de plaies, dans lesquels lesdits exosomes sont obtenus par un procédé comprenant les étapes consistant à :
(a) appliquer une stimulation ultrasonore avec une intensité de 1 W/cm² à une fréquence de 20 kHz pendant une durée de 5 secondes à un milieu contenant des fibroblastes dermiques humains (HDF), le milieu étant l'un quelconque parmi un milieu pour cellules souches épithéliales, un milieu pour cellules souches dermiques, un milieu pour cellules souches adipeuses, un milieu pour cellules souches mésenchymateuses, ou un milieu pour cellules souches embryonnaires ; et appliquer une stimulation ultrasonore, avec une intensité de 5 W/cm² à une fréquence de 20 kHz pendant une durée de 10 minutes, à un milieu pour cellules souches embryonnaires ne contenant pas les HDF ;
(b) mélanger le milieu contenant les HDF, obtenu à l'étape (a), avec le milieu ne contenant pas les HDF, obtenu à l'étape (a) ;
(c) cultiver le mélange obtenu à l'étape (b) pendant 1 jour ; et isoler des exosomes à partir du mélange.
